# EUROPÄISCHE PATENTSCHRIFT

(11) **EP 0 264 666 B1**
(45) Veröffentlichungstag und Bekanntmachung des Hinweises auf die Patenterteilung: **27.01.1993**
(21) Anmeldenummer: 87114138.8
(22) Anmeldetag: 28.09.1987
(51) Int. Cl.: H01L 41/08, A61N 1/36

(54) **Aktivitätssensor für einen Herzschrittmacher**
Activity sensor for a cardiac pace-maker
Détecteur d'activité pour un stimulateur cardiaque

(30) Priorität: 30.09.1986 DE 3633255
(43) Veröffentlichungstag der Anmeldung: 27.04.1988
(73) Patentinhaber: Pacesetter AB, 171 95 Solna (SE)
(72) Erfinder: Nilsson, Kenth, S-184 00 Akersberga (SE)
(74) Vertreter: Lettström, Richard Wilhelm

(56) Entgegenhaltungen:
- US-A- 3 239 678
- US-A- 3 456 134

## Beschreibung

Die Erfindung betrifft einen Aktivitätssensor für einen Herzschrittmacher. Es sind bereits piezoelektrische Aktivitätssensoren des Biegetyps bekannt, (Firmendruckschrift: "VIBRIT, (eingetragenes Warenzeichen), Piezokeramik von Siemens", Bestell-Nr. N-281/5035, 1981,S. 19 u. 20). Diese Sensoren bestehen aus zwei aufeinandergeklebten piezoelektrischen Scheiben, die üblicherweise gegensinnig polarisiert sind. Bei dieser Struktur sind die beiden Aussenflächen des Piezoelementes mit je einer Elektrode versehen. Ausserdem liegt zwischen den beiden Scheiben eine Mittenelektrode.

Wenn diese Struktur gebogen wird, entsteht eine elektrische Spannung zwischen den beiden äusseren Elektroden, die über Anschlüsse abgegriffen werden kann und als Mass für die auf das Piezoelement einwirkende Aktivität dient. Die Spannung setzt sich zusammen aus einer Spannung über der oberen und einer über der unteren Piezokeramikscheibe. In einer der beiden Piezokeramikscheiben liegen elektrische Spannung und Polarisationsrichtung gleichsinnig, in der anderen dagegen gegensinnig. Diese Verhältnisse werden verständlich, wenn man berücksichtigt, dass beim Biegen des Piezoelementes in der oberen Piezokeramikscheibe beispielsweise eine mechanische Spannung und, da die beiden Scheiben zusammengesetzt sind, dadurch in der unteren Scheibe ein Druck, d.h. eine negative mechanische Spannung, entsteht.

Es ist bekannt, dass Piezokeramik durch grosse mechanische Belastungen depolarisiert werden kann. Die Piezokeramik verliert dabei ganz oder zumindest teilweise ihre Empfindlichkeit als Aktivitätssensor und wird für diese Anwendung unbrauchbar. Die Depolarisierung geschieht dadurch, dass die Keramik selbst bei einer grossen mechanischen Belastung eine entsprechend grosse elektrische Spannung erzeugt, die der Polarisation entgegengerichtet ist. Bei der oben beschriebenen Geometrie ist es unerheblich, in welche Richtung die Struktur gebogen wird. Eine der erzeugten elektrischen Spannungen wird immer der Polarisation in einer der Scheiben entgegengerichtet sein.

Alternativ können bei einem derartigen Aktivitätssensor des Biegetyps beide Piezokeramikscheiben in gleicher Richtung polarisiert sein. In diesem Fall muss die Mittenelektrode, d.h. die Elektrode zwischen den beiden Piezokeramikscheiben, für einen Anschluss zugänglich sein. Üblicherweise werden die beiden äusseren Elektroden zusammengeschaltet und die Spannung zwischen diesen und der Mittenelektrode abgegriffen. Dabei wird jedoch das Ausgangssignal gegenüber einem Sensor mit gegensinnig polarisierten Piezokeramikscheiben halbiert. Gleichzeitig wird die Kapazität viermal so gross, so dass die elektrische Energie konstant bleibt. Das Risiko für eine mechanische Depolarisierung ist für beide Strukturen gleich gross, unabhängig davon, in welche Richtung die Strukturen gebogen werden.

Wenn die Struktur stets nur in einer Richtung gebogen werden soll, kann beispielsweise nur eine der beiden Piezokeramikschichten ausgenutzt werden. Dadurch wird das Risiko für eine Depolarisierung vermieden, notwendig ist jedoch, dass die Mittenelektrode angeschlossen werden muss, was oft eine Komplikation darstellt, da diese oft schwer zugänglich ist. Auch hier ergibt sich nur das halbe Ausgangssignal und darüber hinaus nur die halbe elektrische Energie.

Weiterhin besteht die Möglichkeit, dass das Piezoelement nur aus einer einzigen, beidseitig kontaktierten Piezokeramikscheibe besteht und diese beispielsweise auf die Innenwand eines Herzschrittmachergehäuses aufgeklebt ist. Hierbei treten entsprechende Nachteile wie bei den beiden vorangehend beschriebenen Strukturen auf.

Ein weiterer Nachteil all dieser Strukturen ergibt sich insbesondere dann, wenn sie beispielsweise durch Verkleben an einer Oberfläche befestigt werden sollen. Dabei kann es sich beispielsweise um die Innenoberfläche eines Herzschrittmachergehäuses handeln. Solange diese Oberfläche leitend ist, besteht die Möglichkeit, auch die Elektrode des Piezoelementes, die an dieser Oberfläche anliegt, über das Gehäuse elektrisch anzuschliessen. Wenn diese Elektrode jedoch von dem Gehäuse isoliert werden soll oder wenn die Oberfläche, an der das Piezoelement angeklebt ist, selbst aus Isoliermaterial besteht, ergeben sich Kontaktierungschwierigkeiten.

Der vorliegenden Erfindung liegt die Aufgabe zugrunde, einen Aktivitätssensor der eingangs genannten Art so zu verbessern, dass auch bei grossen mechanischen Belastungen eine Depolarisierung in allen Teilen des Piezoelementes sicher vermieden wird. Weiterhin liegt der Erfindung die Aufgabe zugrunde, die Kontaktierung eines derartigen Sensors unabhängig davon, an welchen Oberflächen er angebracht werden soll, wesentlich zu vereinfachen.

Diese Aufgabe wird erfindungsgemäss durch die Merkmale des Anspruchs 1 gelöst.

Aus der US-A-3 239 678 ist ein piezoelektrischer Energiewandler mit nebeneinander angeordneten und gegensinnig polarisierten Keramikteilen bekannt, bei dem die Keramikteile als freischwingende Biegebalken angeordnet sind. Die Keramikteile können alternativ auch an einem Gehäuse befestigt sein und werden dann auf Druck beansprucht.

Bei der erfindungsgemäßen Struktur des Aktivitätssensors werden, wenn dieser einer äußeren Aktivität ausgesetzt wird, in den entsprechenden Keramikteilen elektrische Spannungen erzeugt, die in jedem dieser Teile die gleiche Richtung haben wie die dort vorherrschende Polarisation. Das Risiko für eine Depolarisierung ist dadurch ausgeschaltet, auch wenn dieser Aktivitätssensor starken äusseren Kräften oder Schlägen ausgesetzt ist. Ein weiterer Vorteil besteht darin, dass sich die beiden Anschlüsse, zwischen denen die elektrische Spannung abgegriffen wird, auf einer Seite des Piezoelementes befinden. Dadurch ist es ohne Probleme möglich, dieses Piezoelement beispielsweise an der Innenseite eines Herzschrittmachergehäuses anzukleben, das dabei direkt als Träger für das Piezoelement dient. Das Piezoelement kann ohne Schwierigkeiten auch von dem Herzschrittmachergehäuse isoliert werden oder überhaupt direkt auf elektrisch isolierenden Flächen appliziert werden.

Bei Verwendung von zwei Keramikteilen entspricht die Ausgangsspannung zwischen den Anschlüssen der Summe der Spannungen in den beiden Keramikteilen.

Ein weiterer Vorteil des erfindungsgemässen Aktivitätssensors liegt in seiner höheren Empfindlichkeit. Um das näher zu erläutern, ist beispielhaft die Empfindlichkeit als die Ausgangsspannung an den Anschlüssen dividiert durch die auf den Sensor einwirkende Kraft definiert. Für das Beispiel sei weiterhin angenommen, dass ein Keramikteil die Form eines "Brettes" hat. Es sei weiter angenommen, dass die mechanische Biegesteifigkeit für dieses Keramikteil "eins" sei. Werden zwei derartige Keramikteile nebeneinander angeordnet, wie es bei dem erfindungsgemässen Aktivitätssensor vorgesehen ist, so hat diese Struktur die Biegesteifigkeit 2.

Werden anstelle - wie beim Stand der Technik - zwei derartige Keramikteile aufeinander gelegt und zusammengeleimt, so ergibt sich für diese Struktur eine Biegesteifigkeit mit dem Wert 8.

Dieser Vergleich zeigt, dass bei der erfindungsgemässen Struktur eine um den Faktor 4 kleinere Kraft notwendig ist, um die Struktur genauso weit zu biegen wie die gemäss dem Stand der Technik. Bei entsprechender Formgebung kann daher die erfindungsgemässe Struktur sehr viel empfindlicher gemacht werden als die bisher verwendeten Strukturen.

Der erfindungsgemässe Aktivitätssensor vereinigt damit die Kombination folgender Vorteile:
a) hohe Empfindlichkeit,
b) leicht zu kontaktieren und
c) unempfindlich selbst gegen hohe mechanische Kräfte.

Insbesondere die letzte Eigenschaft, die sicherstellt, dass das Piezoelement nicht depolarisiert wird und damit seine Empfindlichkeit verliert, ist besonders wertvoll bei der Verwendung eines derartigen Sensors in einem Herzschrittmacher, für den extrem hohe Zuverlässigkeit notwendig ist.

In einer konstruktiv vorteilhaften Ausgestaltung ist vorgesehen, dass das Piezoelement aus einer einzigen Platte besteht, deren Oberflächen mit Elektroden beschichtet sind, wobei auf einer Seite zwischen diesen ein isolierender Spalt besteht. Vorteilhafterweise sollte dabei der Spalt mindestens doppelt so breit sein wie die Dicke des Piezoelementes. Die Herstellung des Aktivitätssensors wird auf diese Art und Weise wesentlich vereinfacht. Zunächst wird eine piezokeramische Platte beidseitig mit einer durchgehenden elektrischen Schicht versehen und anschliessend wird im Bereich des Spaltes die elektrische Schicht abgetragen, was beispielsweise durch Schleifen oder Ätzen geschehen kann. Anschliessend können die Keramikteile durch Anlegen einer äusseren Spannung einfach polarisiert werden. Durch die gewählte Spaltbreite wird ein Durchschlagen zwischen den Anschlüssen dabei sicher verhindert.

In Weiterbildung der Erfindung ist vorgesehen, dass die Biegesteifigkeit des Keramikteils an die des Herzschrittmachergehäuses angepasst ist, an dem er befestigt ist. Dadurch wird die Kraftübertragung zwischen dem Herzschrittmachergehäuse und dem Sensor optimiert und damit dessen hohe Empfindlichkeit maximal ausgenutzt. Optimale Verhältnisse ergeben sich, wenn die Grenzschicht zwischen Herzschrittmachergehäuse und Keramikteil dabei die "neutrale Fläche" bildet, d.h. die Fläche, die beim Biegen des Sensors ihre Abmessungen beibehält. Das ganze Keramikteil wird dann je nach Biegerichtung gedehnt oder gestaucht. Piezokeramik und Herzschrittmachergehäuse bilden so zusammen das für einen Biegewandler notwendige Laminat.

In einer besonders vorteilhaften Weiterbildung der Erfindung ist vorgesehen, dass die in dem Aktivitätssensor erzeugte elektrische Spannung zusätzlich in bekannter Weise zum Betreiben von elektronischen Schaltungen verwendbar ist. Insbesondere bei einem implantierbaren Herzschrittmacher kann dadurch die notwendige Batteriekapazität gesenkt und damit vorteilhaft das Volumen des Herzschrittmachers vermindert werden. Aus der US-A- 3,456,134 ist beispielsweise bekannt, wie die Ausgangsspannung eines Piezoelementes zur Spannungsversorgung der Herzschrittmacherelektronik herangezogen werden kann. Vorteilhafterweise wird gemäss der vorliegenden Erfindung die erzeugte Spannung gleich doppelt verwertet, einmal zur Frequenzsteuerung und einmal zur Spannungsversorgung.

Anhand von 5 FIG werden im folgenden ein bekannter und drei Ausführungsformen des erfindungsgemässen Aktivitätssensors schematisch und nicht massstabsgetreu dargestellt. Dabei zeigen
- FIG 1: im Schnitt einen bekannten Aktivitätssensor,
- FIG 2: ein erstes Ausführungsbeispiel des erfindungsgemässen Aktivitätssensors,
- FIG 3: einen weiteren erfindungsgemässen Sensor bei Verwendung in einem Herzschrittmacher,
- FIG 4: diesen Sensor in Draufsicht und
- FIG 5: eine weitere Ausführungsform eines erfindungsgemässen Aktivitätssensors.

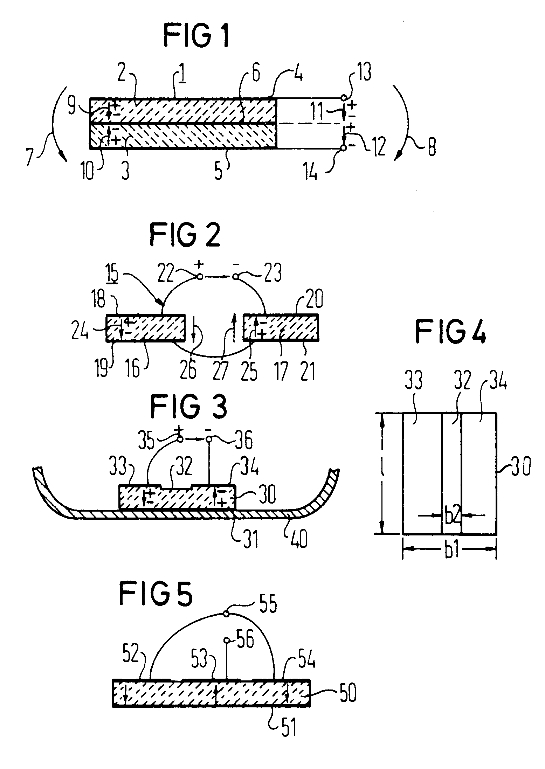

In FIG 1 besteht der Aktivitätssensor (1) aus zwei Piezokeramikteilen (2 und 3), die aufeinander geklebt sind. Weiterhin sind zwei äussere Elektroden (4 und 5) sowie eine Mittenelektrode (6) vorgesehen. Die Pfeile (9 bzw. 10) zeigen die Polarisationsrichtung in den Piezokeramikteilen (2 und 3) an. Die Pfeile (7 und 8) zeigen beispielhaft die Biegerichtung an. Die Pfeile (11 bzw. 12) zeigen die Richtung der erzeugten elektrischen Spannung an. Zwischen den Anschlüssen (13 und 14) kann die elektrische Spannung abgegriffen werden, die der Summe der in den einzelnen Keramikteilen erzeugten Spannung entspricht.

Dieser schematischen FIG 1 ist noch einmal deutlich zu entnehmen, dass in einem Keramikteil - in diesem Fall im Keramikteil (3) - die erzeugte elektrische Spannung, wie sie durch den Pfeil (12) angedeutet ist, der Polarisationsrichtung, wie sie durch den Pfeil (10) angedeutet ist, entgegengerichtet ist, wodurch die Gefahr einer Depolarisierung nicht auszuschliessen ist.

In FIG 2 ist ein erster erfindungsgemässer Aktivitätssensor (15) dargestellt, bei dem zwei Keramikteile (16 bzw. 17) nebeneinander angeordnet sind. Die Oberflächen der beiden Keramikteile sind wiederum mit Elektroden (18, 19 bzw. 20, 21) beschichtet. Die Elektroden (19 und 21) sind elektrisch miteinander verbunden. Die Elektroden (18 bzw. 20) sind mit Anschlüssen (22 bzw. 23) verbunden. Wiederum ist durch Pfeile (24 bzw. 25) die Polarisationsrichtung in den beiden Keramikteilen angedeutet, ebenso wie durch die Pfeile (26 bzw. 27) die elektrische Spannungsrichtung angegeben ist. Wie bereits dieser FIG 2 zu entnehmen ist, liegt die elektrische Spannung stets gleichgerichtet mit der Polarisaitonsrichtung, so dass eine Depolarisation vermieden wird. Da die unteren Elektroden (19 bzw. 21) nur elektrisch miteinander verbunden, aber nicht für einen Anschluss zugänglich sein müssen, kann diese Struktur auf der Unterseite leicht isoliert oder auch auf eine Isolierfläche aufgeklebt werden, ohne die Wirkungsweise des Sensors zu beeinflussen. Auf die Darstellung eines Trägers, der zusammen mit den Keramikteilen das Laminat eines Biegewandlers bildet, wurde in dieser FIG 2 verzichtet.

In FIG 3 ist eine andere Ausführungsform des erfindungsgemässen Aktivitätssensors dargestellt. Dieser besteht aus einem Piezokeramikplättchen (30), das zwei parallele Streifen mit entgegengesetzter Polarisierung aufweist. Die Unterseite dieses Piezoelementes ist mit einer durchgehenden Elektrode (31) verbunden; auf der Oberseite sind zwei durch einen Spalt (32) getrennte Elektroden (33 bzw. 34) vorgesehen. Diese Elektroden sind mit Anschlüssen (35 bzw. 36) versehen, zwischen denen die elektrische Spannung abgegriffen werden kann. Das Piezoelement ist auf die Innenwand eines hier nur teilweise angedeuteten Herzschrittmachergehäuses (40) geklebt. Auf die Darstellung der weiteren bekannten Bestandteile des Herzschrittmachers ist der Übersichtlichkeit halber verzichtet worden.

In FIG 4 ist in Draufsicht noch einmal der Aktivitätssensor allein dargestellt. Für die Verwendung in einem Herzschrittmacher kann dabei folgende Dimensionierung vorgesehen sein:

| | |
|---|---|
| Breite b1 des piezoelektrischen Plättchens ca. | 5 mm, |
| Länge 1 des Plättchens | 10 mm, |
| Breite b2 des Spaltes | 1 mm, |
| Dicke des Plättchens | 0,4 mm, |
| Dicke der Elektrodenschichten | 0,003mm. |

In FIG 5 ist ein weiteres Ausführungsbeispiel eines Aktivitätssensors dargestellt, bei dem das Piezoelement drei Bereiche unterschiedlicher Polarisierung aufweist. Wiederum ist ein einziges Keramikplättchen (50) verwendet, das auf der Unterseite mit einer Elektrode (51) und auf der Oberseite mit drei Elektroden (52-54) versehen ist. Die beiden Elektroden (52,54) gehören zu Bereichen gleichgerichteter Polarisation und sind beide mit einem Anschluss (55) verbunden. Der Bereich unter der Elektrode (53) ist in entgegengesetzter Richtung polarisiert. Diese Elektrode (53) ist mit einem weiteren Anschluss (56) verbunden. Zwischen diesen beiden Anschlüssen wird dann wiederum die elektrische Spannung abgegriffen.

## Patentansprüche

1. Aktivitätssenor für einen Herzschrittmacher, welcher Aktivitätssensor als aus einem Laminat bestehender Biegewandler ausgebildet ist, wobei ein auf je zwei entgegengesetzten Seiten mit Elektroden (18-21;31,33,34;51-54) kontaktierte und zwischen diesen jeweiligen Seiten gegensinning polarisierte Keramikteile (16,17) aufweisendes Piezoelement (30,50) und ein Herzschrittmachergehäuse (40) zusammen das für den Biegewandler notwendige Laminat bilden, wobei die Keramikteile (16,17) nebeneinander angeordnet mit je einer der genannten Seiten vollflächig an der Innenseite des Herzschrittmachergehäuses (40) befestigt sind, und wobei die Elektroden (18-21; 31,33,34; 51-54) auf einer Seite des Piezoelements (30,50) elektrisch miteinander verbunden und auf der anderen Seite mit elektrischen Anschlüssen (22,23;35,36;55,56) verbunden sind, an denen eine in dem Piezoelement (30,50) erzeugte elektrische Spannung als Maß für die auf dieses einwirkende Aktivität zur Frequenzsteuerung des Herzschrittmachers abgreifbar ist.

2. Aktivitätssensor nach Anspruch 1, **dadurch gekennzeichnet**, daß das Piezoelement (30,50) durch Kleben mit dem Herzschrittmachergehäuse (40) verbunden ist.

3. Aktivitätssensor nach Anspruch 1 oder 2, **dadurch gekennzeichnet**, daß das Piezoelement (30,50) aus einer einzigen Platte besteht, deren Seiten mit den Elektroden (31,33,34; 51-54) beschichtet sind, wobei auf einer Seite zwischen den Elektroden ein isolierender Spalt (32) besteht.

4. Aktivitätssensor nach Anspruch 3, **dadurch gekennzeichnet**, daß die Breite (b2) des Spaltes (32) mindestens zweimal so groß ist wie die Dicke der Platte.

5. Aktivitätssensor nach einem der vorangehenden Ansprüche, **dadurch gekennzeichnet**, daß die Biegesteifigkeit des Piezoelements (30,50) an die des Herzschrittmachergehäuses (40) angepaßt ist.

6. Aktivitätssensor nach einem der vorangehenden Ansprüche, **dadurch gekennzeichnet**, daß die erzeugte elektrische Spannung zusätzlich in bekannter Weise zum Betreiben von elektronischen Schaltungen verwendbar ist.

## Claims

1. Activity sensor for a pacemaker, which activity sensor is constructed as a bending transducer comprising a laminate, it being the case that a piezoelectric element (30, 50) which is contacted in each case on two opposite sides by electrodes (18-21; 31, 33, 34; 51-54) and has between these respective sides oppositely polarised ceramic parts (16, 17) forms, together with a pacemaker housing (40), the laminate required for the bending transducer, the ceramic parts (16, 17) being mounted, arranged adjacent to one another, on the inside of the pacemaker housing (40) with full-area coverage of in each case one of the said sides, and the electrodes (18-21; 31, 33, 34; 51-54) being connected on one side of the piezoelectric element (30, 50) to one another and on the other side to electrical terminals (22, 23; 35, 36; 55, 56) at which an electric voltage generated in the piezoelectric element (30, 50) can be tapped as a measure of the activity affecting said elements for the frequency control of the pacemaker.

2. Activity sensor according to Claim 1, characterised in that the piezoelectric element (30, 50) is connected to the pacemaker housing (40) by bonding.

3. Activity sensor according to Claim 1 or 2, characterised in that the piezoelectric element (30, 50) comprises a single plate the sides of which are coated with the electrodes (31, 33, 34; 51-54), an insulating gap (32) existing on one side between the electrodes.

4. Activity sensor according to Claim 3, characterised in that the width (b2) of the gap (32) is at least twice as large as the thickness of the plate.

5. Activity sensor according to one of the preceding claims, characterised in that the bending stiffness of the piezoelectric element (30, 50) is matched to that of the pacemaker housing (40).

6. Activity sensor according to one of the preceding claims, characterised in that the electric voltage generated can additionally be used in a known way to operate electronic circuits.

## Revendications

1. Détecteur d'activité pour un stimulateur cardiaque, qui est agencé sous la forme d'un transducteur à flexion constitué par un stratifié, et dans lequel un élément piézoélectrique (30,50), qui comporte des pièces en céramique (16,17) qui sont en contact, sur deux faces respectivement opposées, avec des électrodes (18-21;31,33,34;51-54) et sont polarisées en des sens opposés, et un boîtier (40) du stimulateur cardiaque forment conjointement le stratifié nécessaire pour le transducteur à flexion, et dans lequel les pièces en céramique (16,17) sont fixées sur la face intérieure du boîtier (40) du stimulateur cardiaque en étant disposées côte-à-côte, sur toute la surface avec respectivement l'une desdites faces, et dans lequel les électrodes (18-21;31,33,34;51-54) sont raccordées électriquement entre elles sur une face de l'élément piézoélectrique (30,50) et sont raccordées, sur l'autre face, à des bornes électriques (22,23;35,36;55,56), sur lesquelles une tension électrique produite dans l'élément piézoélectrique (30,50) peut être prélevée en tant que mesure de l'activité, qui agit sur cet élément, pour la commande de la fréquence du stimulateur cardiaque.

2. Détecteur d'activité suivant la revendication 1, caractérisé par le fait que l'élément piézoélectrique (30, 50) est réuni par collage au boîtier (40) du stimulateur cardiaque.

3. Détecteur d'activité suivant la revendication 1 ou 2, caractérisé par le fait que l'élément piézoélectrique (30,50) est constitué par une seule plaque, dont les faces sont recouvertes par les électrodes (31,33,34;51-54), une fente isolante (32) étant formée sur une face entre les électrodes.

4. Détecteur d'activité suivant la revendication 3, caractérisé par le fait que la largeur (b2) de la fente (32) est égale au moins au double de l'épaisseur de la plaque.

5. Détecteur d'activité suivant l'une des revendications précédentes, caractérisé par le fait que la résistance à la flexion de l'élément piézoélectrique (30,50) est adaptée à celle du boîtier (40) du stimulateur cardiaque.

6. Détecteur d'activité suivant l'une des revendications précédentes, caractérisé par le fait que la tension électrique produite peut être utilisée en supplément, de façon connue, pour faire fonctionner des circuits électroniques.
